# EUROPEAN PATENT SPECIFICATION

(11) **EP 1 354 945 B1**
(45) Date of publication and mention of the grant of the patent: **12.09.2007**
(21) Application number: 01271887.0
(22) Date of filing: 26.12.2001
(51) Int. Cl.: C12N 15/09, C12N 15/10, C12Q 1/68

(54) **METHOD OF IDENTIFYING WITHIN A RODENT A DNA ENCODING PHYSIOLOGICALLY ACTIVE POLYPEPTIDE**
VERFAHREN ZUR IDENTIFIZIERUNG VON EIN PHYSIOLOGISCH AKTIVES POLYPEPTID KODIERENDER DNA IN EINEM NAGETIER
PROCEDE D'IDENTIFICATION D'ADN CODANT POUR UN POLYPEPTIDE ACTIF AU PLAN PHYSIOLOGIQUE DANS UN CORPS DE RONGEUR

(30) Priority: 26.12.2000 JP 2000396316
(43) Date of publication of application: 22.10.2003
(73) Proprietor: CHUGAI SEIYAKU KABUSHIKI KAISHA, Tokyo, 115-8543 (JP)
(72) Inventor: HABU, Kiyoshi, Gotenba-shi, Shizuoka 412-8513 (JP); JISHAGE, Kou-ichi, Gotenba-shi, Shizuoka 412-8513 (JP); SUZUKI, Hiroshi, Hokkaido, 080-0028 (JP)
(74) Representative: Bassil, Nicholas Charles
(86) International application number: PCT/JP2001/011481
(87) International publication number: WO 2002/052001

(56) References cited:
- US-A- 4 675 285
- MAHAN M J ET AL: "SELECTION OF BACTERIAL VIRULENCE GENES THAT ARE SPECIFICALLY INDUCED IN HOST TISSUES" SCIENCE, AMERICAN ASSOCIATION FOR THE ADVANCEMENT OF SCIENCE,, US, vol. 259, 29 January 1993 (1993-01-29), pages 686-688, XP002030831 ISSN: 0036-8075
- SCHREIBER JOHN R ET AL: "Variable region-identical monoclonal antibodies of different IgG subclass directed to Pseudomonas aeruginosa lipopolysaccharide O-specific side chain function differently" JOURNAL OF INFECTIOUS DISEASES, vol. 167, no. 1, 1993, pages 221-226, XP009027285 ISSN: 0022-1899
- LERMAN M I ET AL: "Cloning and characterization of putative genes that specify sensitivity to neoplastic transformation by tumor promoters." INTERNATIONAL JOURNAL OF CANCER. JOURNAL INTERNATIONAL DU CANCER. UNITED STATES 15 FEB 1986, vol. 37, no. 2, 15 February 1986 (1986-02-15), pages 293-302, XP009027284 ISSN: 0020-7136
- MANCINI M ET AL: "DNA-based immunization against the envelope proteins of the hepatitis B virus" JOURNAL OF BIOTECHNOLOGY, ELSEVIER SCIENCE PUBLISHERS, AMSTERDAM, NL, vol. 44, no. 1, 26 January 1996 (1996-01-26), pages 47-57, XP004036848 ISSN: 0168-1656
- GRIFANTINI RENATA ET AL: "Multi-plasmid DNA vaccination avoids antigenic competition and enhances immunogenicity of a poorly immunogenic plasmid" EUROPEAN JOURNAL OF IMMUNOLOGY, vol. 28, no. 4, April 1998 (1998-04), pages 1225-1232, XP009032675 ISSN: 0014-2980
- BARRY M A ET AL: "PROTECTION AGAINST MYCOPLASMA INFECTION USING EXPRESSION-LIBRARY IMMUNIZATION" NATURE, MACMILLAN JOURNALS LTD. LONDON, GB, vol. 377, no. 6550, 19 October 1995 (1995-10-19), pages 632-635, XP002009749 ISSN: 0028-0836
- PIEDRAFITA D ET AL: "Protective immune responses induced by vaccination with an expression genomic library of Leishmania major." JOURNAL OF IMMUNOLOGY (BALTIMORE, MD.: 1950) UNITED STATES 1 AUG 1999, vol. 163, no. 3, 1 August 1999 (1999-08-01), pages 1467-1472, XP002285895 ISSN: 0022-1767
- LOIRAT D ET AL: "Multiepitopic HLA-A*0201-restricted immune response against hepatitis B surface antigen after DNA-based immunization" JOURNAL OF IMMUNOLOGY 15 OCT 2000 UNITED STATES, vol. 165, no. 8, 15 October 2000 (2000-10-15), pages 4748-4755, XP002243973 ISSN: 0022-1767
- BARRY M A ET AL: "Production of monoclonal antibodies by genetic immunization" BIOTECHNIQUES, EATON PUBLISHING, NATICK, US, vol. 16, no. 4, April 1994 (1994-04), pages 616-618,620, XP002130819 ISSN: 0736-6205
- DONNELLY J J ET AL: "IMMUNIZATION WITH DNA" JOURNAL OF IMMUNOLOGICAL METHODS, ELSEVIER SCIENCE PUBLISHERS B.V.,AMSTERDAM, NL, vol. 176, no. 2, 21 December 1994 (1994-12-21), pages 145-152, XP001005665 ISSN: 0022-1759
- RIZZUTO, G. ET AL.: 'Efficient and regulated erythropoietin production by naked DNA injection and muscle electroporation' PROC. NATL. ACAD. SCI. USA vol. 96, 1999, pages 6417 - 6422, XP002168199
- NIWA, H. ET AL.: 'Efficient selection for high-expression transfectants with a novel eukaryotic vector' GENE vol. 108, no. 2, 1991, pages 193 - 199, XP000569330
- IZUMI SAITO ET AL.: 'The protocol series idenshi donyu & hatsugen kaiseki jikkenho', 01 September 1997, YODOSHA CO., LTD. XP002950155 Separative volume, Experimental Medicine * page 63 - page 69 *

## Description

### Technical Field

The present invention relates to a method for identifying within a rodent a DNA encoding a physiologically active polypeptide, and a kit utilized therein.

### Background Art

Conventionally, protein purification has been an essential method for identifying a DNA encoding a physiologically active polypeptide from tissues or cells. Alternatively, the nucleotide sequence of a gene can be predicted by bioinformatics, and the real function of the gene can be identified by generating knockout mice or transgenic mice, and analyzing them.

However, some human tumor cells that produce physiologically active substances cannot be maintained *in vitro* or in immunodeficient animals (for example, nude mice or SCID mice), or they change their characteristics during passage, and sometimes, stop producing physiologically active substances. In such cases, the conventional approaches are incapable of identifying the physiologically active substances.

In addition, when such a physiologically active substance is identified from a pool of multiple genes, it is unpractical to generate knockout animals or transgenic animals for the respective genes in order to detect their physiological activities because this requires a lot of time and effort.

### Disclosure of the Invention

The present invention was accomplished under such circumstances, and its objective is to provide a method for effectively identifying a DNA that has a physiological activity from a pool of multiple DNAs.

In order to solve the above issues, the present inventors examined whether it is possible to identify within mammals, a DNA that has a particular physiological activity from a pool of multiple DNAs.

Specifically, genes encoding EPO or IL-6 were separately inserted into vectors comprising the cytomegalovirus enhancer and chicken β-actin promoter sequences capable of strongly expressing inserted genes in mammalian cells. The resulting vectors were coinjected with an empty vector into mice through the tail vein, and the effects of EPO and IL-6 were examined.

Surprisingly, the result showed that the effects of EPO and IL-6 do not interfere with each other and can be independently detected within mice. The effects of EPO and IL-6 were also detected when the empty vector was present in the vector pool at 1000 folds more than the EPO gene expression vector or the IL-6 gene expression vector.

Thus, the present inventors found that it is possible to independently detect within a rodent a physiological effect of a particular DNA contained in a DNA pool containing various DNAs in various quantities. This finding suggests that it is possible to identify a DNA conferring a particular physiological effect by sequentially fractionating a pool of various DNAs in various quantities, using as the index, the particular physiological effect *in vivo.* Since such a method uses a rodent body, it has the advantage of enabling the identification of a DNA conferring a physiological effect even when, for example, the cells producing the physiologically active substance cannot be maintained *in vitro* or in immunodeficient animals, or when the cells change their characteristics during passage. Furthermore, the method of screening has the additional advantage of saving much time and effort as required in conventional screenings such as those utilizing transgenic and knockout mice.

The present invention relates to a method for effectively identifying within a rodent a DNA that has a physiological activity. More specifically, the present invention provides:
(1) a method for identifying a DNA encoding a physiologically active polypeptide, comprising the steps of:
   (a) introducing DNAs encoding at least two kinds of polypeptides into a rodent, and expressing the DNAs in said rodent,
   (b) detecting a physiological change in said rodent,
   (c) fractionating the DNAs used in step (a) when a physiological change is detected in step (b), and detecting said physiological change in said rodent as in step (b) for each fractionated DNA, and
   (d) identifying a fractionated DNA that has said physiological change in said rodent;
(2) the method according to (1), which further comprises the step of:
   (e) repeating (i) fractionating the DNAs, (ii) detecting said physiological change in said rodent for each fractionated DNA, and (iii) identifying a fractionated DNA that confers said physiological change in said rodent until a DNA is identified that confers said physiological change in said rodent;
(3) the method according to (1) or (2), wherein the DNAs are intravenously injected into the rodent;
(4) the method according to (3), wherein the intravenous injection is carried out through the tail vein;
(5) the method according to (1) or (2), wherein DNAs encoding at least 10 kinds of polypeptides are introduced into the rodent in step (a);
(6) the method according to (1) or (2), wherein DNAs encoding at least 100 kinds of polypeptides are introduced into the rodent in step (a);
(7) the method according to (1) or (2), wherein DNAs encoding at least 1000 kinds of polypeptides are introduced into the rodent in the step (a) ;
(8) the method according to (1) or (2), wherein the DNA is derived from an animal cell, an animal tissue, or an animal cell line;
(9) the method according to (1) or (2), wherein the DNA is derived from a tumor cell, a tumor tissue, or a tumor cell line;
(10) the method according to (1) or (2), wherein the total amount of DNA injected into the mammal at one time is 100 µg or less;
(11) the method according to (1) or (2), wherein the total amount of DNA injected into the rodent at one time is 10 µg or less.

The present invention provides a method for identifying within a rodent a DNA encoding a physiologically active polypeptide.

First, in the method of the present invention, DNAs encoding at least two kinds of polypeptides are introduced into a rodent, and expressed in the rodent *in vivo* ((1) step (a)).

There is no particular limitation on DNA introduced into rodents in the present invention; a DNA encoding any polypeptide whose physiological activity one desires to identify can be used. For example, a DNA derived from an animal cell, an animal tissue, or an animal cell line can be suitably used. Specifically, any DNA can be used depending on the purpose of the experiment; for example, a DNA derived from T cells, hematopoietic cells, or hematopoietic stem cells can be used when one wants to identify a DNA associated with a blood disease or an autoimmune disease, or a DNA derived from a tumor cell, a tumor tissue or a tumor cell line can be used when one wants to identify a DNA participating in tumor development.

The present inventors discovered that even when multiple DNAs encoding different kinds of physiologically active polypeptides are expressed simultaneously within a rodent, it is possible to independently detect the physiological activity of each. Therefore, the characteristic of the present invention is that DNAs encoding at least two kinds of polypeptides are introduced into a mammal. It is possible to introduce into a rodent DNAs encoding 10 or more (for example, 100 or more, 1000 or more) kinds of polypeptides.

There is no particular limitation on the single dose of DNA introduced into a rodent as long as the physiological activity of the expression product of the DNA is detectable; however, it is preferably 100 µg or less. Since the present invention enabled the detection of an expressed polypeptide even when the total DNA amount injected into the rodent was 10 µg or less, it is possible to inject a DNA at a dose of 10 µg or less. The injection of a DNA into a rodent can be repeated several times if necessary.

A DNA to be introduced into a rodent is normally inserted into a mammalian cell expression vector. There is no particular limitation on mammalian cell expression vectors as long as they ensure the expression of the inserted DNA within cells. For example, a vector utilizing for example the chicken β-actin promoter (for example, pCAGGS; Niwa H. et al., Gene 108: 193-200 (1991)), the CMV promoter (for example, pCMV-Script; Stratagene, pcDNA3.1/His; Invitrogen), the EF-1α promoter (for example, pEF1/His ; Invitrogen), or the SRα promoter (for example, pME18SFL3; Maruyama K. and Sugano S., Gene 138: 171 (1994)) can be used. In particular, the pCAGGS vector comprising the cytomegalovirus enhancer and chicken β-actin promoter sequences can be suitably used in the present invention. When a mammalian cell expression vector is introduced into a rodent *in vivo,* the vector may be also mixed with a polymer, liposome, PEI (polyethylenimine),or such to improve DNA expression efficiency. Alternatively, a virus vector represented by a known retrovirus vector or adenovirus vector may be used instead of mammalian cell expression vectors.

There is no particular limitation on rodents used in the present invention; for example, rodents such as rats, mice, and such can be suitably used. DNA can be introduced into such rodents by a variety of methods such as intravenous injection, intramuscular injection, oral administration, subcutaneous or intracutaneous injection, intranasal administration, direct injection into an organ such as the liver or heart, etc. For rodents such as rats and mice, injection through the tail vein may be suitably used. The expression efficiency can be improved by using the Gene Gun (Bio-Rad) or electrical stimulation when DNAs are introduced.

Next, in the present invention, a physiological change is detected in the above rodent (step (b)).

There is no limitation on the physiological change to be detected, and it can include a change in blood cells (for example, the change in the number of erythrocytes, lymphocytes, or platelets), a change in the biochemical values in blood (for example, the change in the GOT or GTP value, or the change in the level of glucose, calcium, or phosphate), or a change in the urea level in urine. A physiological change of interest may be decided in advance by the persons conducting the experiment. Alternatively, a physiological change of interest may be found after introducing the DNA into rodents.

Next, in the present invention, when a physiological change is detected in step (b), the DNA used in step (a) is fractionated, and each fractionated DNA is used to detect the physiological change in the rodent (step (c)).

The above step can be carried out as follows. When *E. coli* is used as a host to construct a library of DNA that has been introduced into a rodent *in vivo,* a pool of *E. coli* cells corresponding to the DNA fraction with which the physiological activity was detected is identified and then spread on plates to clone individual *E. coli* cells. The cloned *E. coli* cells are fractionated, and from each fraction, a DNA (vector) is prepared. Each prepared DNA fraction is introduced into the rodent to detect the physiological change of interest.

Next in the present invention, a DNA fraction that induces the physiological change in the rodent is identified (step (d)).

When the DNA fraction identified in the above step comprises multiple DNAs, according to the need, it is possible to repeat (i) fractionating the DNA further, (ii) examining whether each fractionated DNA causes the physiological change in the rodent, and (iii) identifying a DNA that causes the physiological change in the rodent, until a DNA that causes the physiological change is identified (step (e)). The nucleotide sequence of the identified DNA may be determined depending on the need of subsequent experiments.

### Brief Description of the Drawings

Fig. 1 is a graph showing the effect of the expression of genes introduced into mice.
Fig. 2 is a photograph showing the expression of the introduced genes in mouse liver. Six µg of polyA⁺ RNA for each was used in the northern blot analysis.
Fig. 3 is a graph showing the effect of EPO expressed within mice.
Fig. 4 is a graph showing the effect of IL-6 expressed within mice.

### Best Mode for Carrying out the Invention

This invention will be explained in detail below with reference to examples, but it is not to be construed as being limited thereto.

### [Example 1] A method of screening for a DNA utilizing mouse bodies

The present inventors searched for a method in which when a pool of 100 or 1000 genes with unknown functions is simultaneously introduced into a mouse, and some physiological effect is observed, a gene having the physiological activity is identified by repeating the step of fractionating the original gene pool which is followed by reintroducing them into mice.

The method utilizes the pCAGGS vector developed by Miyazaki et al. (Niwa H. et al., Gene 108: 193-200 (1991)) to overexpress a gene within mice. The pCAGGS vector in which the cytomegalovirus enhancer and the chicken β-actin promoter are combined, is capable of strongly expressing an inserted gene in mammalian cells. The present inventors incorporated a gene of interest into the vector, and resuspended it in a solution accompanying an *in vivo* gene transfection kit (commercial name: TransIt In Vivo Gene Delivery System, TaKaRa), and injected the vector into mice through the tail vein.

### (1) Analysis of the expression of a DNA within mice

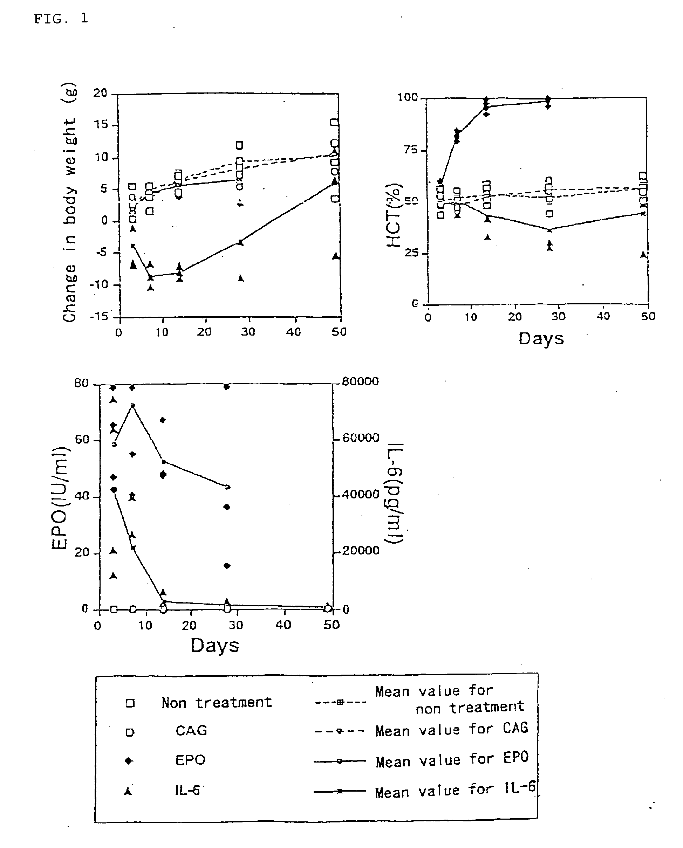

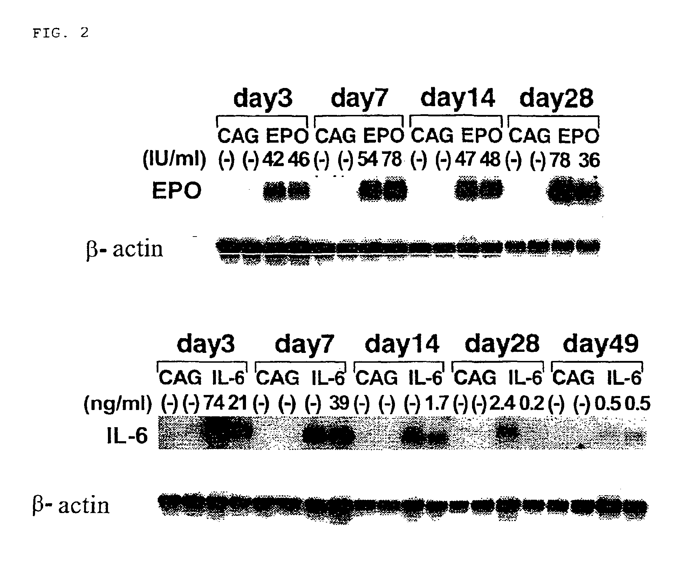

When a vector containing the GFP gene was injected (normally injected at 10 µg vector per mouse), a strong GFP expression was detected in the liver of mice six days after injection. Similarly, when a vector containing the mouse EPO gene or mouse IL-6 gene was injected, the corresponding cytokine was detected in blood (Fig. 1), and the mRNA was detected in the liver (Fig. 2).

### (2) Application to screening

In screenings where usually a large number of genes must be screened, it is unpractical to inject each vector (clone) containing a gene into mice one by one considering that the a lot of time and effort should be required. Therefore, the present inventors thought that in an effective screening, the effect of a single vector should be detected even when a mixture of at least 100 kinds of vectors is simultaneously injected into a mouse. Thus, the present inventors examined how much DNA can be diluted (even when vectors other than that carrying the gene of interest is present at an excess amount) in order to detect the effect of the EPO gene and IL-6 gene by mixing vectors containing these two genes. The experiment was carried out as follows (Table 1).

**Table 1**

| Group | Estimated clone number | CAG | EPO | IL-6 |
|---|---|---|---|---|
| 1 | - | 100 µg | 0 | 0 |
| 2 | 1000 | 99.8 µg | 0.1 | 0.1 |
| 3 | 100 | 98 µg | 1 | 1 |
| 4 | | 80 µg | 10 | 10 |
| 5 | | 9.8 µg | 0.1 | 0.1 |
| 6 | | GFP 98 µg | 1 | 1 |
| 7 | Non treatment | - | - | - |

A group of ICR male mice aged 12 weeks (n=4 or 5) was used for each group, and samples were collected on the day following the injection (day 1) and after one week (day 7). The clone numbers indicate the estimated number of clones mixed at the time of the screening. For example, in group 2, the EPO vector or IL-6 vector represents 1/1000 of total DNA. Thus, if the effect of EPO or IL-6 is detected in the experiment, it indicates that 0.1 µg per clone may be sufficient to detect the effect. It also indicates that the effect of a gene of interest can be detected without the interference of various contaminants even when there was 99.8 µg of vectors other than the vector of interest (that is in the presence of 1000 such vectors).

Group 1 is for determining the background level of the effect of DNA injection. Group 4, in which the EPO and IL-6 vectors are injected at a dose normally used to inject the respective vector alone into mice, is designed to examine whether an effect that was not observed when each vector was used alone might be induced or whether the effects of each might interfere with each other when the two vectors are mixed. Group 5 is a control in which the DNA is injected at 10 µg in total, the dose normally injected into mice. Group 6 is a control that estimates the effect of the overexpression of a protein other than the protein of interest (GFP in this case). Group 7 is a control where the mice have not undergone any treatment.

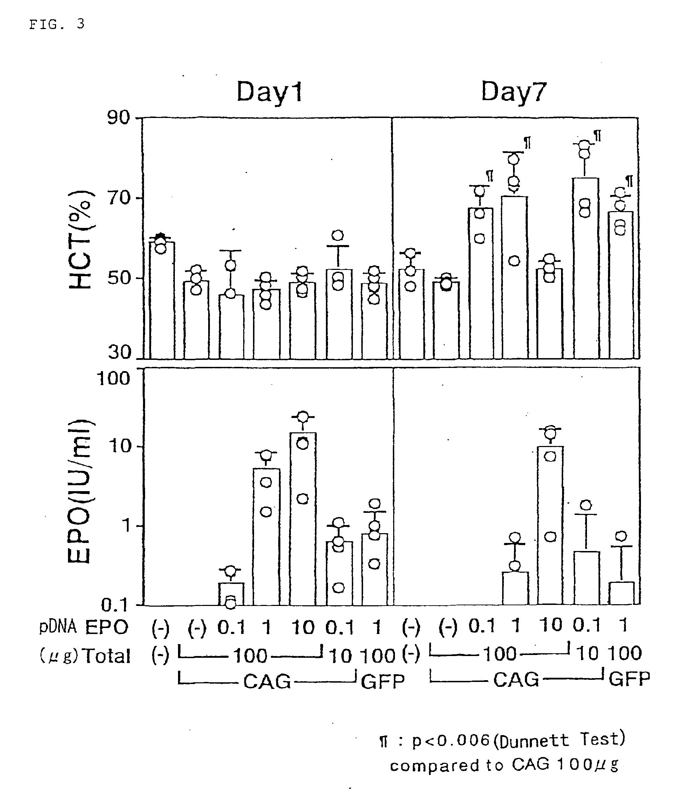

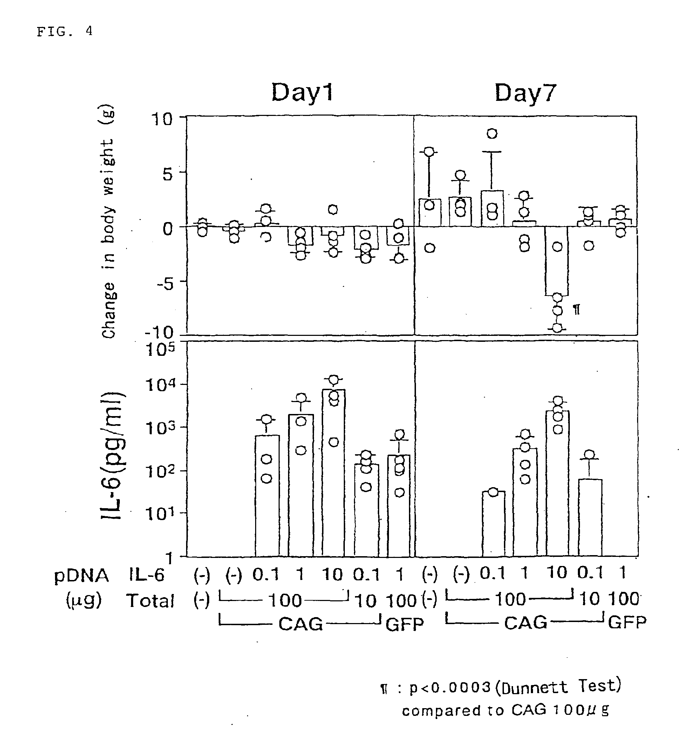

The results are shown in Fig. 3 and 4. The result of injecting each vector at 10 µg (Fig. 1) showed that at day 7, there was an increase in the number of erythrocytes in the group injected with the EPO vector, and a significant decrease in body weight in the group injected with the IL-6 vector (IL-6 is a causative gene of cachexia) . Using these effects of the respective genes as indexes, the data were interpreted as follows.

In group 3 injected with a pool of 100 clones (CAG 98 µg, EPO 1 µg, and IL-6 1 µg) that is estimated to be used normally in screenings for genes with unknown functions, there was a significant increase in the HCT value compared to the control group (group 1), and a tendency to have suppressed body weight increases. Almost the same effects were observed in group 5 (CAG 9.8 µg, EPO 0.1 µg, and IL-6 0.1 µg), indicating that it is possible to use DNA at 10 µg in total and decrease the dose to 0.1 µg per clone.

The above results confirmed that it is possible to detect the effect of each single gene without mutual interference even when a pool of a variety of genes is simultaneously introduced. Furthermore, it is thought that the method of the *in vivo* screening of the present invention can even work with a pool of 1,000 clones if there is a suitable system that reflects the effect of the target gene.

### Industrial Applicability

Conventionally, the purification of active substances (proteins), from, for example, the culture supernatant of cells producing them, has been the general method. When the nucleotide sequence is known for a gene with an unknown function, a recombinant protein of the gene can be prepared in CHO cells or such, and used to immunize animals such as mice to prepare an antibody against the protein. Such a recombinant protein and antibody can be used in combination to analyze the gene function. It is also possible to predict the nucleotide sequence of a gene using a computer, and use it to prepare a transgenic animal, which is then used to analyze gene function. Recently, a series of saturation mutagenesis methods have enabled the preparation of a library of mutant mice, with which, for example, a causative gene (mutation) for a disease of interest can be identified from mice having the disease. However, all such methods require a lot of time and effort for protein purification, preparation of antibodies or animals, etc. Although it is possible to identify a gene that is expressed in a cell-specific manner by using a DNA chip or such, it is difficult to narrow it down as a target of drug development or functional analysis by relying solely on the expression profile. The method of the present invention is particularly effective when (1) no appropriate assay system is available *in vitro,* but only *in vivo*, or (2) the cells or tissue producing the active substance is limited, or when there is a possibility that the activity may get lost during passage. Moreover, the method of the present invention enables one to isolate the functional gene faster than conventional methods by eliminating the steps for protein purification, or preparation of animals, etc. It is also possible to obtain antibodies against multiple antigens at once by repeating the method of the present invention. Thus, it is possible, for example, to construct a library of antibodies against a tumor by introducing repetitively a pool of genes that are expressed in tumor cells. Thus, it is also possible to select an antibody effective against the tumor cells from the library, and identify an antigen to which the antibody binds.

## Claims

1. A method for identifying a DNA encoding a physiologically active polypeptide, comprising the steps of:
(a) introducing DNAs encoding at least two kinds of polypeptides into a rodent, and expressing the DNAs in said rodent,
(b) detecting a physiological change in said rodent,
(c) fractionating the DNAs used in step (a) when a physiological change is detected in step (b), and detecting said physiological change in said rodent as in step (b) for each fractionated DNA, and
(d) identifying a fractionated DNA that has said physiological change in said rodent.

2. The method according to claim 1, which further comprises the step of:
(e) repeating (i) fractionating the DNAs, (ii) detecting said physiological change in said rodent for each fractionated DNA, and (iii) identifying a fractionated DNA that confers said physiological change in said rodent until a DNA is identified that confers said physiological change in said rodent.

3. The method according to claim 1 or 2, wherein the DNAs are intravenously injected into the rodent.

4. The method according to claim 3, wherein the intravenous injection is carried out through the tail vein.

5. The method according to claim 1 or 2, wherein DNAs encoding at least 10 kinds of polypeptides are introduced into the rodent in step (a).

6. The method according to claim 1 or 2, wherein DNAs encoding at least 100 kinds of polypeptides are introduced into the rodent in step (a).

7. The method according to claim 1 or 2, wherein DNAs encoding at least 1000 kinds of polypeptides are introduced into the rodent in the step (a).

8. The method according to claim 1 or 2, wherein the DNA is derived from an animal cell, an animal tissue, or an animal cell line.

9. The method according to claim 1 or 2, wherein the DNA is derived from a tumor cell, a tumor tissue, or a tumor cell line.

10. The method according to claim 1 or 2, wherein the total amount of DNA injected into the rodent at one time is 100 µg or less.

11. The method according to claim 1 or 2, wherein the total amount of DNA injected into the rodent at one time is 10 µg or less.

## Patentansprüche

1. Ein Verfahren zum Identifizieren einer ein physiologisch aktives Polypeptid codierenden DNA, aufweisend die Schritte:
(a) Einführen von zumindest zwei Arten von Polypeptide codierende DNAs in ein Nagetier und Expression der DNAs in dem Nagetier,
(b) Auffinden einer physiologischen Veränderung bei dem Nagetier,
(c) Fraktionieren der bei Schritt (a) verwendeten DNAs wenn eine physiologische Veränderung bei Schritt (b) aufgefunden wird, und Auffinden der physiologischen Veränderung bei dem Nagetier gemäß Schritt (b) für jede fraktionierte DNA, und
(d) Identifizieren einer fraktionierten DNA, die die physiologische Änderung bei dem Nagetier bedingt.

2. Das Verfahren nach Anspruch 1, welches ferner die Schritte aufweist:
(e) Wiederholen (i) des Fraktionierens der DNAs, (ii) des Auffindens der physiologischen Veränderung bei dem Nagetier für jede fraktionierte DNA und (iii) des Identifizierens einer fraktionierten DNA, die die physiologische Veränderung bei dem Nagetier bedingt, bis eine DNA identifiziert wird, die die physiologische Veränderung bei dem Nagetier bedingt.

3. Das Verfahren nach Anspruch 1 oder 2, wobei die DNAs intravenös in das Nagetier injiziert werden.

4. Das Verfahren nach Anspruch 3, wobei die intravenöse Injektion durch die Schwanzvene ausgeführt wird.

5. Das Verfahren nach Anspruch 1 oder 2, wobei zumindest 10 Arten von Polypeptide codierende DNAs bei Schritt (a) in das Nagetier eingeführt werden.

6. Das Verfahren nach Anspruch 1 oder 2, wobei zumindest 100 Arten von Polypeptide codierende DNAs bei Schritt (a) in das Nagetier eingeführt werden.

7. Das Verfahren nach Anspruch 1 oder 2, wobei zumindest 1000 Arten von Polypeptide codierende DNAs bei Schritt (a) in das Nagetier eingeführt werden.

8. Das Verfahren nach Anspruch 1 oder 2, wobei die DNA aus einer Tierzelle, einem Tiergewebe oder einer Zelllinie eines Tieres gewonnen wird.

9. Das Verfahren nach Anspruch 1 oder 2, wobei die DNA aus einer Tumorzelle, einem Tumorgewebe oder einer Zelllinie eines Tumors gewonnen wird.

10. Das Verfahren nach Anspruch 1 oder 2, wobei die Gesamtmenge der in das Nagetier auf einmal injizierten DNA 100 µg oder weniger beträgt.

11. Das Verfahren nach Anspruch 1 oder 2, wobei die Gesamtmenge der in das Nagetier auf einmal injizierten DNA 10 µg oder weniger beträgt.

## Revendications

1. Méthode pour identifier un ADN codant pour un polypeptide physiologiquement actif, comprenant les étapes suivantes :
(a) introduction des ADN codant pour au moins deux types de polypeptides dans un rongeur, et expression des ADN chez ledit rongeur,
(b) détection d'une variation physiologique chez ledit rongeur,
(c) fractionnement des ADN utilisés dans l'étape (a) lorsqu'une variation physiologique est détectée dans l'étape (b), et détection de ladite variation physiologique chez ledit rongeur comme dans l'étape (b) pour chaque ADN fractionné, et
(d) identification d'un ADN fractionné qui présente ladite variation physiologique chez ledit rongeur.

2. Méthode suivant la revendication 1, qui comprend en outre l'étape suivante :
(a) répétition (i) du fractionnement des ADN, (ii) détection de ladite variation physiologique chez ledit rongeur pour chaque ADN fractionné, et (iii) identification d'un ADN fractionné qui confère ladite variation physiologique chez ledit rongeur jusqu'à l'identification d'un ADN qui confère ladite variation physiologique chez ledit rongeur.

3. Méthode suivant la revendication 1 ou 2, dans laquelle les ADN sont injectés au rongeur par voie intraveineuse.

4. Méthode suivant la revendication 3, dans laquelle l'injection intraveineuse est effectuée dans la veine de la queue.

5. Méthode suivant la revendication 1 ou 2, dans laquelle les ADN codant pour au moins 10 types de polypeptides sont introduits dans le rongeur dans l'étape (a).

6. Méthode suivant la revendication 1 ou 2, dans laquelle les ADN codant pour au moins 100 types de polypeptides sont introduits dans le rongeur dans l'étape (a).

7. Méthode suivant la revendication 1 ou 2, dans laquelle les ADN codant pour au moins 1000 types de polypeptides sont introduits dans le rongeur dans l'étape (a).

8. Méthode suivant la revendication 1 ou 2, dans laquelle l'ADN est dérivé d'une cellule animale, d'un tissu d'un animal ou d'une lignée de cellules animales.

9. Méthode suivant la revendication 1 ou 2, dans lequel l'ADN est dérivé d'une cellule tumorale, d'un tissu tumoral ou d'une lignée de cellules tumorales.

10. Méthode suivant la revendication 1 ou 2, dans laquelle la quantité totale d'ADN injectée en une fois dans le rongeur est égale ou inférieure à 100 µg.

11. Méthode suivant la revendication 1 ou 2, dans laquelle la quantité totale d'ADN injectée en une fois dans le rongeur est égale ou inférieure à 10 µg.
